# EUROPÄISCHE PATENTSCHRIFT

(11) **EP 2 329 074 B1**
(45) Veröffentlichungstag und Bekanntmachung des Hinweises auf die Patenterteilung: **19.10.2016**
(21) Anmeldenummer: 09775616.7
(22) Anmeldetag: 27.08.2009
(51) Int. Cl.: D06M 15/03, A61K 8/73, D06M 101/06

(54) **VERFAHREN ZUR BEHANDLUNG CELLULOSISCHER FORMKÖRPER**
METHOD FOR TREATING CELLULOSE MOLDINGS
PROCÉDÉ DE TRAITEMENT DE CORPS MOULÉS CELLULOSIQUES

(30) Priorität: 22.09.2008 AT 14712008
(43) Veröffentlichungstag der Anmeldung: 08.06.2011
(73) Patentinhaber: Lenzing Aktiengesellschaft, 4860 Lenzing (AT)
(72) Erfinder: REDLINGER, Sigrid, A-4860 Lenzing (AT); RICHARDT, Werner, A-4880 St. Georgen (AT); FIRGO, Heinrich, A-4840 Voecklabruck (AT)
(74) Vertreter: Nemec, Harald
(86) Internationale Anmeldenummer: PCT/AT2009/000334
(87) Internationale Veröffentlichungsnummer: WO 2010/031091

(56) Entgegenhaltungen:
- AT-U2- 8 388
- DE-A1-102004 049 282
- DATABASE WPI Week 200382 Thomson Scientific, London, GB; AN 2003-881274 XP002554734 & JP 2003 096671 A (MARUMASA YG) 3. April 2003 (2003-04-03)

## Beschreibung

Die Erfindung betrifft ein Verfahren zur Behandlung eines cellulosischen Formkörpers, insbesondere von Cellulosefasern für Textilzwecke oder Nonwovens.

Insbesondere betrifft die Erfindung ein Verfahren zur Modifizierung der Eigenschaften von cellulosischen Formkörpern mittels Chitosan.

Chitin und Chitosan sind natürliche, biologisch abbaubare, nicht toxische, nicht allergene, bioaktive und biokompatible Polymere und in ihrer Struktur der Cellulose ähnlich. Chitin wird aus den Hüllen von Krustentieren gewonnen, einem Abfallprodukt der Krabben- und Shrimpsindustrie. Das weltweite Interesse bezüglich der Verwendungsmöglichkeiten von Chitin hat, da es neben Cellulose als zweitgrößte Ressourcenquelle für natürliche Polysaccharide gesehen wird, in den letzten Jahren enorm zugenommen.

Chitosan besteht aus Poly-(1,4)-2-Amino-2-Deoxy-Beta-D-Glucose und wird durch Deacetylierung von Chitin (Poly-(1,4)-2-Acetamid-2-Deoxy-Beta-D-Glucose) hergestellt. Aus Löslichkeitsgründen - Chitin ist unlöslich in Wasser, organischen Lösungsmitteln, verdünnten Säuren und Laugen - hat Chitosan, welches in verdünnten Säuren, wässrigem Methanol und Glycerin löslich ist, die weitaus größere Bedeutung.

Anwendungsgebiete für Chitin und Chitosan sind in der Biotechnologie zur Immobilisierung von Zellen und Enzymen, in der Medizin zur Wundbehandlung, im Lebensmittelbereich als Nahrungsmittelzusatz- und Konservierungsstoff, in der Landwirtschaft zur Samenkonservierung, in Abwassersystemen als Flockungsmittel und Chelatbildner mit Schwermetallen.

Allerdings muss für die meisten Anwendungsgebiete eine Modifizierung des Chitins/Chitosans durchgeführt werden, um die Löslichkeit in wässrigen Systemen zu verbessern.

Der Einsatz von Chitosan in der Textilindustrie gliedert sich in drei Anwendungsgebiete:
- Herstellung von 100% Chitosanfasern bzw. Herstellung von "Man Made Fibres" mit Inkorporation von Chitosan
- Finishing und Coating von Textilfasern
- Prozesshilfsstoffe für die Textilindustrie

Chitosanfasern finden ihre Anwendung im medizinischen Bereich z.B. als Wundabdeckungen und chirurgische Nahtfäden aufgrund der antibakteriellen Eigenschaften und der Wachstumshemmung auf pathogene Keime. Chitin bzw. Chitosan können durch körpereigene Fermente enzymatisch bzw. hydrolytisch abgebaut werden und sind daher resorbierbare Fasern. Die Wirkung dieser Naturpolymere bei der Wundheilung besteht in der allmählichen Abgabe von N-Acetyl-Glukosamin, der mucopolysacchariden Organisation des Collagens sowie der positiven Beeinflussung des Gewebewachstums im Verlauf der Wundheilung (EP 0 077 098, US 4309534, JP81/112937, JP84/116418 und zahlreiche mehr).

Der Nachteil von Fasern aus 100% Chitosan ist allerdings, dass sie eine geringe Trockenfestigkeit besitzen (Chitosanfasern der Fa. Innovative Technology Ltd., Winsford, England: Titer 0,25 tex; Faserfestigkeit konditioniert 9 cN/tex, Faserdehnung konditioniert 12,4%; Chitosanfasern der Fa. Korea Chitosan Co. LTD: Faserfestigkeit konditioniert 15 cN/tex; Faserdehnung konditioniert 26%), ausgesprochen spröde sind und die Nassfestigkeit nur 30% der Trockenfestigkeit beträgt. Daher werden entweder Chitosanfasern anderen Man-Made Fasern beigemischt bzw. bereits beim Herstellungsprozess von z.B. Viskosefasern Chitosan in die Spinnmasse beigegeben.

Kommerziell erhältlich sind Viskosefasern mit inkorporiertem Chitin/Chitosan (im folgenden: "chitosaninkorporierte Viskosefasern") z.B. unter dem Handelsnamen Crabyon (Fa. Omikenshi Co) und Chitopoly (Fa. Fuji Spinning Co). Hergestellt werden diese Fasern beispielsweise, indem Chitosan oder acetyliertes Chitosan in Pulverform mit einer Korngröße kleiner 10 µm in einer Menge von 0,5 bis 2 Gew.% in Wasser dispergiert wird und der Viskosespinnlösung zugegeben wird (US 5,320,903). Dann werden nach dem herkömmlichen Viskose- oder auch Polynosicverfahren Fasern hergestellt.

Weitere Herstellungsverfahren für chitosaninkorporierte Viskosefasern sind in der US-A 5,756,111 (aufwendige Vor- und Nachlöseprozesse bei Tieftemperatur, um alkalische Chitin-Chitosan-Lösungen für die Zugabe zur Viskoselösung zu erhalten), der US-A 5,622,666 (Zugabe von mikrokristallinem Chitosan und einem wasser- und/oder alkalilöslichen natürlichen Polymer, z.B. Natriumalginat, welches ionische Bindungen zum Chitosan bilden kann, als Dispersion zur Viskosespinnlösung) und PCT/FI90/00292 bzw. FI 78127 (Zugabe von mikrokristallinem Chitosan zur Spinnmasse) beschrieben.

AT 8388 U beschreibt die Verwendung einer Cellulosefaser, welche ein Chitosan oder ein Chitosansalz inkorporiert und/oder an der Oberfläche der Faser aufweist, in einem nichtgewebten Textil und/oder einem saugfähigen Hygieneprodukt.

Die chitosaninkorporierten Viskosefasern haben eine erhöhte Farbstoffaffinität, ein erhöhtes Wasserrückhaltevermögen, antifungizide und geruchshemmende Eigenschaften, allerdings auch die für Viskosefasern bekannte geringe Nassfestigkeit. Da Chitosan das Wachstum von für die Haut schädlichen Bakterien verhindert und allergische Effekte eliminiert, sind z.B. Gewebe aus Chitopoly für Dermatitispatienten besonders geeignet.

Der Nachteil aller beschriebenen Verfahren besteht darin, daß die so erhaltenen Fasern feinste Chitosanpartikeln enthalten, da das Chitosan in der Spinnmasse nicht löslich ist.

Die Sekundäragglomeration des Chitosans in der Spinnmasse bzw. die inhomogene Verteilung führt zu einer Verschlechterung der Spinneigenschaften, das Spinnen von Fasern mit niedrigen Titern ist extrem schwierig. Aus diesem Grund kann auch die Menge an inkorporiertem Chitosan nicht erhöht werden, da damit sofort ein Verlust an textilen Daten eintritt bzw. es bereits beim Spinnen zu zahlreichen Fadenbrüchen kommt. Weiters kommt es, da Chitosan in Säuren löslich ist, im Spinnbad zu Chitosanverlusten. Zur Inkorporation von Chitosan sind zusätzliche aufwendige Schritte notwendig.

Um die Wirkung des Chitosans im Endprodukt sicherzustellen, muss zudem eine Menge von zumindest ca. 10 Gew.% Chitosan in die Fasern inkorporiert werden, da nur dann genügend Chitosan an der Faseroberfläche ist. Das im Inneren der Fasern inkorporierte Chitosan ist nämlich unzugänglich und daher nicht wirksam.

In der Folge wurde auch versucht, in lösungsmittelgesponnene Cellulosefasern, die nach dem Aminoxidverfahren hergestellt werden (sogenannte "Lyocell-Fasern"), Chitosan zu inkorporieren, insbesonders wegen der hohen Nass- und Trockenfestigkeit der Lyocellfasern.

In der DE 195 44 097 wird ein Verfahren zur Herstellung von Formkörpern aus Polysaccharidmischungen durch Auflösen von Cellulose und einem Zweitpolysaccharid in einem organischen, mit Wasser mischbaren Polysaccharidlösungsmittel (bevorzugt NMMO), das auch ein Zweitlösungsmittel enthalten kann, beschrieben.

Weiters wird in der KR-A 9614022 die Herstellung von Chitin-Cellulose-Fasern, genannt "Chitulose", beschrieben, indem Chitin und Cellulose in einem Lösungsmittel aus der Gruppe Dimethylimidazoline/LiCl, Dichloroacetat/Chlorkohlenwasserstoff, Dimethylacetamid/LiCl, N-Methylpyrrolidon/LiCl gelöst werden und nach dem Nassspinnverfahren Garne hergestellt werden. In den Ansprüchen ist NMMO nicht erwähnt.

In der EP-A 0 883 645 wird u.a. die Zugabe von Chitosan zur Lösung als modifizierte Verbindung zur Erhöhung der Geschmeidigkeit von Nahrungsmittelhüllen beansprucht. Die modifizierenden Verbindungen müssen mit der Cellulose/NMMO/Wasser-Lösung mischbar sein.

Die KR-A-2002036398 beschreibt die Inkorporation von aufwendig herzustellenden Chitosan-Derivaten mit quaternären Ammoniumgruppen in Fasern.

In der DE-A 100 07 794 wird die Herstellung von Polymerzusammensetzungen beschrieben, umfassend ein biologisch abbaubares Polymer und ein Material aus Meerespflanzen und/oder Schalen von Meerestieren sowie die Herstellung von Formkörpern daraus. Beansprucht wird auch die Zugabe von Material aus Meerespflanzen, Meerestieren in Pulverform, Pulversuspension oder flüssiger Form zur nach dem Lyocellverfahren hergestellten Celluloselösung. Weiters kann das Material auch nach oder während der Zerkleinerung der trockenen Zellulose zugegeben werden, sowie in jeder Stufe des Herstellungsprozesses. Trotz Zugabe des Additives zeigen die Fasern dieselben textilmechanischen Eigenschaften wie ohne Additiv. In den Beispielen werden nur Lyocell-Fasern beschrieben, welche Braunalgenpulver inkorporiert haben, wobei zur Herstellung der Spinnmasse das Braunalgenmehl, NMMO und Zellstoff und Stabilisator gemischt und auf 94°C erwärmt werden.

Weiters wird im Schlussbericht "Erzeugnisse aus Polysaccharidverbunden" (Taeger, E.; Kramer, H.; Meister, F.; Vorwerg, W.; Radosta, S; TITK - Thüringisches Institut für Textil- und Kunststoff-Forschung, 1997, S.1-47, Report-Nr. FKZ 95/NR 036 F) beschrieben, dass Chitosan in verdünnten organischen oder anorganischen Säuren gelöst wird und danach in wässriger NMMO-Lösung gefällt wird. Man erhält so eine Suspension feiner Chitosankristalle in der Celluloselösung, die dann versponnen wird. Gemäß diesem Dokument verbleibt das Chitosan auch nach dem Auflösen der Cellulose als feine Kriställchen in der Lösung. Dadurch kommt es zu einem mikroheterogenen Zweitphasensystem in der Faser. Die Festigkeit der Faser ist gering (bei 10% Chitosan Faserfestigkeit konditioniert 19,4 cN/tex, Faserdehnung konditioniert 11,5%).

In der WO 04/007818 wird vorgeschlagen, ein in der Spinnlösung lösliches Chitosoniumpolymer (ein Salz des Chitosans mit einer anorganischen oder organischen Säure) durch Zugabe zur Spinnlösung oder einem Vorläufer davon in die Lyocell-Faser zu inkorporieren.

Als Alternative zur Inkorporation besteht die Möglichkeit, textile Flächengebilde im Zuge der Herstellung mit Chitosan auszurüsten. Das Aufbringen von Chitosan auf bereits hergestellte Fasern oder diese enthaltende textile Artikel wird im folgenden auch als "Imprägnierung" bezeichnet. Ein grundsätzliches Problem dabei besteht aber darin, dass das so aufgebrachte Chitosan nicht fixiert ist und relativ schnell ausgewaschen wird, wodurch die positiven Effekte verloren gehen.

Um dieses Problem zu umgehen, wird in der EP 1 243 688 die Verwendung von Chitosan-Nanopartikeln zur Herstellung von Fasern, Garnen, Gewirken und textilen Flächengebilden vorgeschlagen. Unter Nano-Chitosanen werden annähernd sphärische Festkörper verstanden, die einen mittleren Durchmesser im Bereich von 10 bis 300 nm aufweisen und aufgrund des kleinen Teilchendurchmessers zwischen die Fibrillen eingelagert werden. Die Herstellung von Nano-Chitosanen erfolgt durch Sprühtrocknung, Evaporationstechnik oder Entspannung von überkritischen Lösungen.

In der WO 01/32751 wird ein Verfahren zur Herstellung von nanopartikulärem Chitosan für kosmetische und pharmazeutische Zubereitungen mit Teilchendurchmessern von 10 bis 1000 nm beschrieben, bei welchem man den pH-Wert einer wässrigen sauren Chitosanlösung in Gegenwart eines Oberflächenmodifikationsmittels soweit anhebt, dass es zur Ausfällung des Chitosans kommt. Weiters wird in der WO 91/00298 die Herstellung von mikrokristallinen Chitosan-Dispersionen und Pulvern mit Teilchendurchmessern von 0,1 bis 50 µm beschrieben, bei welchem man den pH-Wert einer wässrigen sauren Chitosanlösung so weit anhebt, dass es zur Ausfällung des Chitosans kommt.

Die WO 97/07266 beschreibt die Behandlung einer Lyocellfaser mit einer 0,5%igen essigsauren Chitosanlösung.

In der WO 2004/007818 wird neben der Inkorporation eines Chitosoniumpolymers in Lyocell-Fasern auch die Behandlung von niemals getrockneten Lyocell-Fasern mit der Lösung oder Suspension eines Chitosoniumpolymers beschrieben. Es hat sich gezeigt, dass sich dieses Verfahren nur zur Behandlung von niemals getrockneten Lyocell-Fasern eignet. Der Begriff "niemals getrocknet" bezeichnet dabei den Zustand einer frisch ersponnenen Faser, welche noch keinem Trocknungsschritt unterzogen wurde.

Eine Behandlung von anderen Fasertypen als Lyocell-Fasern im niemals getrockneten Zustand (z.B. Modal-Fasern und Viskose-Fasern) ist mit dem Verfahren gemäß der WO 2004/007818 nicht möglich.

In der Österreichischen Patentanmeldung A 82/2008 (nicht vorveröffentlicht), wird ein Verfahren beschrieben, bei welchem ein cellulosischer Formkörper mit einer alkalischen Dispersion, welche ungelöste Chitosanpartikel enthält, in Kontakt gebracht wird.

Die vorliegende Erfindung stellt sich zur Aufgabe, ein Verfahren zur Behandlung von cellulosischen Formkörpern zur Verfügung zu stellen, welches die vorgenannten Probleme einer Inkorporation von Chitosan in Fasern nicht aufweist und welches für verschiedene cellulosische Fasertypen, im getrockneten wie im niemals getrockneten Zustand, geeignet ist. Das Chitosan soll insbesondere an der Faseroberfläche von Cellulose-Regeneratfasern (Lyocell-Fasern, Modal-Fasern, Viskose-Fasern, Polynosic-Fasern) bevorzugt während des Herstellungsprozesses so fixiert werden, dass das Chitosan am Endprodukt auch nach einer Reihe von Haushaltswäschen noch vorhanden ist.

Diese Aufgabe wird mit einem Verfahren zur Behandlung eines cellulosischen Formkörpers, wobei der Formkörper mit einer sauren Lösung eines Chitosans in Kontakt gebracht wird, und welches dadurch gekennzeichnet ist, dass das Chitosan einen Deacetylierungsgrad von mindestens 80%, einen Stickstoffgehalt von zumindest 7 Gew.%, bevorzugt zumindest 7,5 Gew.%, ein Gewichtsmittel der Molmasse M_{w} (D) von 10 kDa bis 1000 kDa, bevorzugt 10 kDa bis 160 kDa und eine Viskosität in 1 Gew.%-Lösung in 1 Gew.%-Essigsäure bei 25°C von 1000 mPas oder weniger, bevorzugt 400 mPas oder weniger, insbesondere bevorzugt 200 mPas oder weniger, aufweist, gelöst.

Es hat sich überraschenderweise gezeigt, dass ein nachhaltiges Aufbringen von Chitosan auf die Oberfläche cellulosischer Formkörper möglich ist, wenn die Formkörper mit einer sauren Lösung, welche das oben spezifizierte Chitosan enthält, behandelt wird. Insbesondere wurde ein überraschender Zusammenhang zwischen der Viskosität von Chitosan in saurer Lösung und der bei Behandlung des Formkörpers erzielbaren Auflage aufgefunden: Je geringer die Viskosität eines Chitosans in saurer Lösung, umso höher (und zwar deutlich höher) die erreichbare Auflage auf dem Formkörper.

Dadurch können ausreichende Auflagen bei vergleichsweise geringem Einsatz an Chitosan erreicht werden.

Der Begriff "Lösung eines Chitosans" bedeutet, dass das Chitosan in vollständig gelöster Form vorliegt. Dieser Begriff schliesst aber nicht die Anwesenheit weiterer, gegebenenfalls ungelöster Bestandteile in der Behandlungsflüssigkeit aus.

Zum Einsatz im erfindungsgemäßen Verfahren eignen sich Chitosane mit einer Viskosität einer 1%-igen Lösung in 1%iger Essigsäure bei 25 °C von 1000 mPas oder weniger, bevorzugt 400 mPas oder weniger, insbesondere bevorzugt 200 mPas oder weniger, gemessen mit einem Brookfield Viscosimeter bei 30 U/min.

Auch der Deacetylierungsgrad des Chitosans spielt eine Rolle: Je höher der Deacetylierungsgrad, umso besser ist das Chitosan zum Einsatz im erfindungsgemäßen Verfahren geeignet.

Geeignete Chitosane können insbesondere eine Polydispersität (Verhältnis von Gewichtsmittel zu Zahlenmittel des Molekulargewichts) von 2 bis 4 aufweisen.

In der Literatur gibt es keine einheitliche Definition für die Abgrenzung zwischen Chitin und Chitosan.

Für die Zwecke der vorliegenden Erfindung soll der Begriff "Chitin" ein β-1,4-gebundenes Polymer der 2-acetamido-2-deoxy-D-glucose mit einem Deacetylierungsgrad von 0% bedeuten. Weiters bedeutet für die Zwecke der vorliegenden Erfindung der Begriff "Chitosan" ein zumindest teilweise deacetyliertes β-1,4-gebundenes Polymer der 2-acetamido-2-deoxy-D-glucose.

Das erfindungsgemäße Verfahren weist gegenüber den bekannten Verfahren zur Inkorporation von Chitosan den Vorteil auf, dass ein inkorporiertes Chitosan im Inneren des Formkörpers nicht zugänglich ist. Nur Chitosan an der Oberfläche des Formkörpers kann in Kontakt mit der Haut kommen und so seine positive Wirkung entfalten. Um dieselbe Menge Chitosan wie bei der Imprägnierung an der Oberfläche eines Formkörpers zu erreichen, müssen daher bei der Inkorporation wesentlich höhere Chitosanmengen verwendet werden.

Gegenüber der Verwendung von Nano-Chitosan besteht insbesondere ein Vorteil anhand der hohen Herstellungskosten von Nano-Chitosan.

Gegenüber dem in der WO 2004/007818 beschriebenen Verfahren besteht bei erfindungsgemäßen Verfahren der Vorteil, dass die dort beschriebene Imprägnierung mit einer sauren Lösung eines Chitosoniumpolymers bei der Behandlung von niemals getrockneten Viskose-, Modal- oder Polynosicfasern mit nachfolgendem Dämpfen nicht funktioniert. Es werden dabei nur äußerst geringe Chitosanauflagen erzielt, und die Durchführung dieses Verfahrens ist ohne Umbau bestehender Anlagen nicht möglich.

Zudem ist das erfindungsgemäße Verfahren kostengünstiger als das in der WO 2004/007818 beschriebene Verfahren, da bevorzugt billigere Chitosantypen verwendet werden können (siehe weiter unten).

Gemäß einer bevorzugten Ausführungsform des erfindungsgemäßen Verfahrens beträgt der Gehalt der Chitosanpartikel in der Lösung 0, 1 bis 10 Gew.%, besonders bevorzugt 1 bis 4 Gew.%.

Der erfindungsgemäß behandelte Formkörper liegt bevorzugt in Form von Fasern vor. Die Fasern können insbesondere Lyocellfasern, Modalfasern, Polynosicfasern und/oder Viskosefasern sein.

Der Gattungsname "Lyocell" wurde von der BISFA (The International Bureau for the Standardisation of Man Made Fibres) vergeben und steht für Cellulosefasern, die aus Lösungen der Cellulose in einem organischen Lösungsmittel hergestellt werden. Bevorzugt werden als Lösungsmittel tertiäre Aminoxide, insbesondere N-methyl-morpholin-N-oxid (NMMO) eingesetzt. Ein Verfahren zur Herstellung von Lyocellfasern ist z.B. in der US-A 4,246,221 beschrieben.

Viskosefasern sind Fasern, die aus einer alkalischen Lösung des Cellulosexanthogenates (Viskose) durch Ausfällen und Regeneration der Cellulose gewonnen werden.

Modalfasern sind Cellulosefasern, die gemäß Definition der BISFA durch eine hohe Nassreißfestigkeit und einen hohen Nassmodul (die Kraft, die benötigt wird, eine Faser im nassen Zustand um 5% zu dehnen) charakterisiert sind.

Die Fasern können bei der Behandlung mit der Chitosanlösung in bereits getrockneter Form, insbesondere als Bestandteil eines textilen Artikels, bevorzugt eines Garnes, eines Gewebes, eines Gestrickes, eines daraus hergestellten Kleidungsstückes, oder eines Non-Woven-Artikels vorliegen.

Als "bereits getrocknete" Fasern versteht man Fasern, die im Zuge ihres Herstellungsverfahrens bereits zumindest einmal einem Trocknungsschritt unterworfen wurden.

Bevorzugt können die Fasern aber in niemals getrockneter Form vorliegen. Als "niemals getrocknet" wird eine Faser bezeichnet, die im Zuge ihres Herstellungsprozesses noch keinem Trocknungsschritt unterworfen wurde. Die Fasern können insbesondere in Form eines Faservlieses, wie dieses im Zuge des Herstellungsverfahrens von Lyocell-, Viskose-, Modal- und Polynosicstapelfasern als Zwischenprodukt auftritt, vorliegen.

Diese Variante hat den Vorteil, dass die Behandlung ohne die Notwendigkeit apparativer Veränderungen in einer bestehenden Anlage zur Herstellung von Lyocell-, Viskose-, Modal- oder Polynosicfasern implementiert werden kann. Eine Behandlung von niemals getrockneten Viskose-, Modal- oder Polynosicfasern mit Chitosan während des Herstellungsprozesses dieser Fasern war bislang nicht möglich.

Die Fasern können vor der Behandlung eine Restfeuchte von 50% bis 500% aufweisen.

Nach der Behandlung mit der Chitosanlösung kann der Formkörper einer Behandlung mit Heißdampf unterzogen werden. Dadurch kann eine zusätzliche Fixierung des Chitosans auf der Oberfläche des Formkörpers erzielt werden.

Zur Herstellung der Lösung wird bevorzugt Chitosan in einer anorganischen oder organischen Säure aufgelöst.

Die Säure ist bevorzugt aus der Gruppe bestehend aus Mono-, Di- oder Tricarbonsäuren mit 1 bis 30 C-Atomen, bevorzugt Milchsäure, Essigsäure, Ameisensäure, Propionsäure, Glykolsäure, Zitronensäure, Oxalsäure sowie Mischungen daraus ausgewählt.

Es hat sich gezeigt, dass die zur Auflösung des Chitosans notwendige Säuremenge vom Deacetylierungsgrad abhängt.

Die zur Lösung des Chitosans notwendige Säuremenge wird abhängig vom Deacetylierungsgrad des verwendeten Chitosans wie folgt berechnet:

**Tabelle 1**

| Deacetylierungsgrad Chitosan in % | Mol Säure pro g Chitosan |
|---|---|
| 80% | 0,00493 |
| 85% | 0,00525 |
| 90% | 0,00555 |
| 95% | 0,00586 |
| 100% | 0,00617 |

Zur Herstellung der Chitosanlösung werden zur entsprechenden Menge Chitosan unter Rühren die wie oben beschrieben notwendige Säuremenge und Wasser zugegeben und solange gerührt, bis eine klare Lösung vorliegt.

Die so erhaltene Chitosanlösung kann beispielsweise zur kontinuierlichen Behandlung mit einem initialfeuchten, durch z.B. Abpressen auf eine definierte Feuchte von 50% bis 500% eingestellten Cellulose-Regeneratfaservlies in Kontakt gebracht werden. Das Vlies kann z.B. durch Besprühen durchtränkt werden. Dazu kann z.B. ohne Notwendigkeit eines Umbaus vorhandener Produktionsanlagen bei Anlagen zur Herstellung von Viskosefasern und Modalfasern das sogenannte Bleichefeld verwendet werden.

Nach der Imprägnierung kann das Vlies auf eine definierte Feuchte von 50% - 500% abgepresst und die abgepreßte Behandlungsflotte in den Imprägnierkreislauf zurückgeführt werden.

Danach wird das Vlies entweder mit Heißdampf behandelt und anschließend neutral gewaschen oder ohne Heißdampfbehandlung neutral gewaschen, aviviert und getrocknet.

Die Bestimmung der Chitosanauflage erfolgt über Messung des Stickstoff-Gehaltes mittels LECO FP 328 Stickstoffanalyser durch Verglühen der Probe. Mittels FITC (Fluoresceinisothiocyanat)-Färbung der Fasern und anschließende Untersuchung mittels Fluoreszenzmikroskop der Fasern kann die Chitosanverteilung auf der Faseroberfläche beobachtet werden.

In einer weiteren bevorzugten Ausführungsform wird der Formkörper vor oder nach dem Trocknen einer Behandlung mit einem Vernetzungsmittel unterzogen.

Die vorliegende Erfindung betrifft außerdem einen Formkörper, der durch das erfindungsgemäße Verfahren erhältlich ist.

Der erfindungsgemäße Formkörper weist einen Gehalt an Chitosan mit den oben angeführten Spezifikationen auf, wobei das Chitosan im wesentlichen vollständig an der Oberfläche des Formkörpers verteilt ist (und nicht etwa in wesentlicher Menge auch im Inneren des Formkörpers enthalten ist).

Der erfindungsgemäße Formkörper kann insbesondere in Form von Fasern, bevorzugt Lyocellfasern, Modalfasern, Polynosicfasern und/oder Viskosefasern vorliegen.

Ein Charakteristikum der nach dem erfindungsgemäßen Verfahren erhältlichen Formkörper besteht darin, dass das Chitosan filmförmig auf der Oberfläche des Formkörpers verteilt ist.

Der erfindungsgemäße Formkörper weist bevorzugt einen Gehalt an Chitosan von 0,1 Gew.% und mehr, bevorzugt 0,2 Gew.% bis 1 Gew.%, insbesondere bevorzugt 0,4 bis 0,6 Gew.% auf. Es hat sich gezeigt, das insbesondere eine gute antibakterielle Wirkung des erfindungsgemäßen Formkörpers bereits bei geringen Auflagen ab 0,1 Gew.% erreicht wird.

Die vorliegende Erfindung betrifft auch die Verwendung eines erfindungsgemäßen Formkörpers als antibakterielles Produkt, als geruchshemmendes Produkt, als wundheilendes, blutstillendes und blutkoagulationsförderndes Produkt, in Nonwovens-Produkten und/oder als Füllfaser. Bevorzugte Anwendungsgebiete von erfindungsgemäßen chitosanhältigen Cellulose-Regeneratfasern umfassen aufgrund der mild antibakteriellen, geruchshemmenden, hautfreundlichen Eigenschaften körpernah getragene Textilien wie z.B. Unterwäsche oder Socken, Textilien für Menschen mit empfindlicher Haut (Neurodermitis), Bettwäsche und Heimtextilien. Als Füllfaser kann die erfindungsgemäße Faser sowohl alleine als auch in Mischungen mit anderen Fasern, wie z.B. Baumwolle, Polyesterfasern und unmodifizierten Cellulosefasern (z.B. Lyocellfasern) eingesetzt werden.

Insbesondere wurde gefunden, dass erfindungsgemäße Celluloseregeneratfasern bereits ab einer Chitosanauflage von 0,1 % signifikant antibakteriell im Shake Flask Test und zellproliferationsfördernd in der regenerierenden Epidermis wirken (geprüft im porcinen *ex-vivo* Wundheilungsmodell).

Daher betrifft die vorliegende Erfindung in einem weiteren Aspekt auch einen erfindungsgemäßen Formkörper, insbesondere mit einem Gehalt an Chitosan von 0,2 Gew.% bis 1 Gew.%, zur spezifischen Verwendung als wundheilendes, insbesondere die Zellproliferation in der regenerierenden Epidermis förderndes Produkt.

Im folgenden wird die Erfindung durch Beispiele und die Zeichnungen näher erläutert.

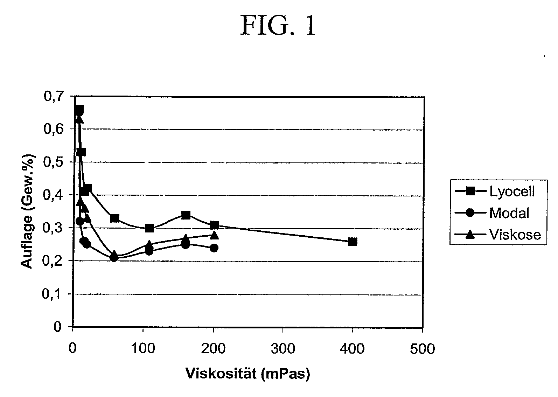

Dabei zeigt die Figur 1 die auf verschiedenen Cellulosefasern unter Anwendung des erfindungsgemäßen Verfahrens erzielten Chitosanauflagen, in Abhängigkeit von der Viskosität des eingesetzten Chitosans.

### BEISPIELE

### Beispiel 1:

Es wurden folgende Chitosantypen zur Behandlung von Cellulosefasern eingesetzt:

**Tabelle 2**

| Firma | Type | BatchNr. | Viskosität mPas | Deacetylierungsgrad % |
|---|---|---|---|---|
| Primex | ChitoClear cg110 | TM2881 | 159 | 82 |
| Primex | ChitoClear cg110 | TM3013 | 108 | 80 |
| Primex | ChitoClear cg110 | TM3089 | 58 | 81 |
| Primex | ChitoClear cg10 | TM2963 | 19 | 81 |
| Primex | ChitoClear fg95LV | TM3091 | 15 | 96 |
| Primex | ChitoClearfg95ULV | TM2875 | 9 | 85 |
| Heppe | 85/200/A1 | | 200 | 85 |
| Heppe | 85/400/A1 | | 400 | 85 |
| Heppe | 90/10/A1 | | 6 | 90 |

Aus diesen Chitosantypen wurde jeweils eine 1 Gew.% Chitosanlösung in wässeriger Milchsäure hergestellt. Die jeweils eingesetzte Menge an Milchsäure wurde gemäß der obigen Tabelle 1 in Abhängigkeit vom Deacetylierungsgrad des eingesetzten Chitosans festgelegt.

Verwendete Faserproben:
1,3 dtex Lyocellfaser NMMO-frei gewaschen, niemals getrocknet
1,3 dtex Modalfaser nicht gebleicht, niemals getrocknet
1,3 dtex Viskosefaser nicht gebleicht, niemals getrocknet

Vorgangsweise bei der Behandlung der Fasern:
Die niemals getrockneten Fasern werden 5 min bei Raumtemperatur im Flottenverhältnis 1 : 10 mit der jeweiligen Chitosanlösung imprägniert, mit 1 bar abgepresst, danach 5 min bei 100°C /100% relativer Feuchtigkeit gedämpft, ausgewaschen und bei 60°C getrocknet.

Die Ergebnisse der Versuche sind in der folgenden Tabelle zusammengefasst.

**Tabelle 3**

| Chitosantype | Chitosanauflage in Gew% | | |
|---|---|---|---|
| Batch Nr./Type | Lyocell | Modal | Viskose |
| TM2881 | 0,34 | 0,25 | 0,27 |
| TM3013 | 0,30 | 0,23 | 0,25 |
| TM3089 | 0,33 | 0,21 | 0,22 |
| TM2963 | 0,42 | 0,25 | 0,33 |
| TM3091 | 0,41 | 0,26 | 0,36 |
| TM2875 | 0,53 | 0,32 | 0,38 |
| 85/200/A1 | 0,31 | 0,24 | 0,28 |
| 85/400/A1 | 0,26 | - | - |
| 90/10/A1 | 0,66 | 0,65 | 0,63 |

Alle so hergestellten Faserproben wurden einer Heißwasserbehandlung im Flottenverhältnis 1 : 20 für 40 min. bei 90°C unterzogen. Die Chitosanauflagen erwiesen sich dabei als permanent.

Die Bestimmung der Chitosanauflage auf der Faser erfolgt mittels Messung des N-Gehaltes (LECO FP 328 Stickstoffanalyser) durch Verglühen der Probe.
Eine FITC(Fluorescein-isothiocyanat)-Färbung der Fasern und anschließende Untersuchung mittels Fluoreszenzmikroskop der Fasern wurde durchgeführt, um die Chitosanverteilung auf der Faseroberfläche zu analysieren.

Aus der nachfolgenden Tabelle ist eindeutig bei allen drei Fasertypen ersichtlich, dass - bei gleicher Gew.%-Konzentration an Chitosan - je niedriger die Viskosität der Lösung ist, desto höher die erzielte Chitosanauflage. Weiters werden die höchsten Chitosanauflagen an der Lyocell-Faser erzielt, da diese im initialfeuchten Zustand offenbar ein besser zugängliches Porensystem aufweist.

**Tabelle 4**

| Viskosität der Chitosanlösung 1%ig in Essigsäure mPas | Chitosanauflage in Gew.% | | |
|---|---|---|---|
| | Lyocell | Modal | Viskose |
| 400 | 0,26 | Kein Versuch | Kein Versuch |
| 200 | 0,31 | 0,24 | 0,28 |
| 159 | 0,34 | 0,25 | 0,27 |
| 108 | 0,30 | 0,23 | 0,25 |
| 58 | 0,33 | 0,21 | 0,22 |
| 19 | 0,42 | 0,25 | 0,33 |
| 15 | 0,41 | 0,26 | 0,36 |
| 9 | 0,53 | 0,32 | 0,38 |
| 6 | 0,66 | 0,42 | 0,47 |

Dieser Zusammenhang ist grafisch in der Figur 1 dargestellt.

### Beispiel 2 - Herstellung einer Lyocellfaser im Produktionsversuch

Es wurde eine Lyocellfaser mit einem Titer von 1,3dtex und 38 mm Schnittlänge mit Chitosan behandelt.

Die gemäß der Lehre der WO 93/19230 hergestellte, niemals getrocknete Lyocellfaser wurde gemäß dem in Beispiel 1 beschriebenen Verfahren mit 1 gew.%iger milchsaurer Chitosanlösung (Chitosantype: TM2875, siehe Tabelle 1) im Flottenverhältnis 1 : 20 auf eine Sollauflage von 0,4 Gew.% Chitosan hin imprägniert, gedämpft, ausgewaschen, aviviert und getrocknet. Aus den so hergestellten Fasern wurden Garne Nm 50 gesponnen und zu einem textilen Flächengebilde (Single jersey Strick) verarbeitet, welches 0,45 % Chitosanauflage aufwies.

Diese Strickproben wurden im Vergleich zu gebleichter Baumwolle und einer nicht mit Chitosan behandelten Lyocellfaser im porcinen ex-vivo Wundheilungsmodell im Universitätsklinikum Hamburg Eppendorf, Zellbiologisches Labor auf den Anteil proliferativer Zellen am Wundrand in der regenerierenden Epidermis und in der unbeteiligten Epidermis geprüft. Es wurden signifikant mehr proliferative Zellen am Wundrand und tendentiell mehr proliferative Zellen in der regenerierenden Epidermis bei der chitosanhältigen Faser gefunden als bei einer nicht behandelten Lyocellfaser und Baumwolle.

## Patentansprüche

1. Verfahren zur Behandlung eines cellulosischen Formkörpers, wobei der Formkörper mit einer sauren Lösung eines Chitosans in Kontakt gebracht wird, **dadurch gekennzeichnet, dass** das Chitosan einen Deacetylierungsgrad von mindestens 80%, einen Stickstoffgehalt von zumindest 7 Gew.%, bevorzugt zumindest 7,5 Gew.%, ein Gewichtsmittel der Molmasse M_{w} (D) von 10 kDa bis 1000 kDa, bevorzugt 10 kDa bis 160 kDa und eine Viskosität in 1 Gew.%-Lösung in 1 Gew.%-Essigsäure bei 25°C von 1000 mPas oder weniger, bevorzugt 400 mPas oder weniger, insbesondere bevorzugt 200 mPas oder weniger, aufweist.

2. Verfahren nach Anspruch 1, **dadurch gekennzeichnet, dass** der Gehalt des Chitosans in der Lösung 0,1 bis 10 Gew.%, bevorzugt 1 bis 4 Gew.% beträgt.

3. Verfahren nach Anspruch 1 oder 2, **dadurch gekennzeichnet, dass** der Formkörper in Form von Fasern vorliegt.

4. Verfahren nach Anspruch 3, **dadurch gekennzeichnet, dass** die Fasern Lyocellfasern, Modalfasern, Polynosicfasern und/oder Viskosefasern sind.

5. Verfahren nach Anspruch 3 oder 4, **dadurch gekennzeichnet, dass** die Fasern bei der Behandlung in bereits getrockneter Form, insbesondere als Bestandteil eines textilen Artikels, bevorzugt eines Garnes, eines Gewebes, eines Gestrickes, eines daraus hergestellten Kleidungsstückes, oder eines Non-Woven-Artikels vorliegen.

6. Verfahren nach Anspruch 3 oder 4, **dadurch gekennzeichnet, dass** die Fasern bei der Behandlung in niemals getrockneter Form vorliegen.

7. Verfahren nach Anspruch 6, **dadurch gekennzeichnet, dass** die Fasern in Form eines Faservlieses vorliegen.

8. Verfahren nach Anspruch 6 oder 7, **dadurch gekennzeichnet, dass** die Fasern vor der Behandlung eine Restfeuchte von 50% bis 500% aufweisen.

9. Verfahren nach einem der vorhergehenden Ansprüche, **dadurch gekennzeichnet, dass** der Formkörper nach der Behandlung mit der Lösung einer Behandlung mit Heißdampf unterzogen wird.

10. Verfahren nach einem der vorhergehenden Ansprüche, **dadurch gekennzeichnet, dass** zur Herstellung der Lösung Chitosan in einer anorganischen oder organischen Säure aufgelöst wird.

11. Verfahren gemäß Anspruch 10, **dadurch gekennzeichnet, dass** die Säure aus der Gruppe bestehend aus Mono-, Di- oder Tricarbonsäuren mit 1 bis 30 C-Atomen, bevorzugt Milchsäure, Essigsäure, Ameisensäure, Propionsäure, Glykolsäure, Zitronensäure, Oxalsäure sowie Mischungen daraus ausgewählt ist.

12. Formkörper, erhältlich durch ein Verfahren gemäß einem der Ansprüche 1 bis 11.

13. Formkörper nach Anspruch 12 in Form von Fasern, bevorzugt Lyocellfasern, Modalfasern, Polynosicfasern und/oder Viskosefasern.

14. Formkörper nach einem der Ansprüche 12 oder 13, **gekennzeichnet durch** einen Gehalt an Chitosan von 0,1 Gew.% und mehr, bevorzugt 0,2 Gew.% bis 1 Gew.%, insbesondere bevorzugt 0,4 bis 0,6 Gew.%.

15. Verwendung eines Formkörpers nach einem der Ansprüche 12 bis 14 als antibakterielles Produkt, als geruchshemmendes Produkt, als wundheilendes, blutstillendes und blutkoagulationsförderndes Produkt, in Nonwovens-Produkten und/oder als Füllfaser.

16. Formkörper nach einem der Ansprüche 12 bis 14, insbesondere mit einem Gehalt an Chitosan von 0,2 Gew.% bis 1 Gew.%, zur spezifischen Verwendung als wundheilendes, insbesondere die Zellproliferation in der regenerierenden Epidermis förderndes Produkt.

## Claims

1. A process for the treatment of a cellulosic moulded body, wherein the moulded body is contacted with an acid solution of a chitosan, **characterized in that** the chitosan has a deacetylation degree of at least 80%, a nitrogen content of at least 7 w%, preferably at least 7.5 w%, a weight average molecular weight M_{w} (D) of 10 kDa to 1000 kDa, preferably 10 kDa to 160 kDa and a viscosity in 1 w% solution in 1 w% acetic acid at 25°C of 1000 mPas or less, preferably 400 mPas or less, particularly preferably 200 mPas or less.

2. A process according to claim 1, **characterized in that** the chitosan content in the solution is 0.1 to 10 w%, preferably 1 to 4 w%.

3. A process according to claim 1 or 2, **characterized in that** the moulded body is present in the form of fibres.

4. A process according to claim 3, **characterized in that** the fibres are Lyocell fibres, Modal fibres, polynosic fibres and/or viscose fibres.

5. A process according to claim 3 or 4, **characterized in that** the fibres intended for treatment are provided in already dried form, in particular as an integral part of a textile article, preferably a yarn, a fabric, a knitted fabric, a piece of clothing produced therewith or a non-woven article.

6. A process according to claim 3 or 4, **characterized in that** the fibres are present in never-dried form during the treatment.

7. A process according to claim 6, **characterized in that** the fibres are present in the form of a fibre fleece.

8. A process according to claim 6 or 7, **characterized in that** the fibres have a residual moisture of 50% to 500% before treatment.

9. A process according to any of the previous claims, **characterized in that** the moulded body is subjected to a treatment with hot vapour after treatment with the solution.

10. A process according to any of the previous claims, **characterized in that** in order to prepare the solution, chitosan is dissolved in an inorganic or organic acid.

11. A process according to claim 10, **characterized in that** the acid is selected from the group consisting of mono-, di- or tricarboxylic acids with 1 to 30 C-atoms, preferably lactic acid, acetic acid, formic acid, propionic acid, glycolic acid, citric acid, oxalic acid as well as mixtures thereof.

12. A moulded body obtainable by a process according to any of the claims 1 to 11.

13. A moulded body according to claim 12 in the form of fibres, preferably Lyocell fibres, Modal fibres, polynosic fibres and/or viscose fibres.

14. A moulded body according to any of the claims 12 or 13, **characterized by** a chitosan content of 0.1 w% and more, preferably 0.2 w% to 1 w%, particularly preferably 0.4 to 0.6 w%.

15. The use of a moulded body according to any of the claims 12 to 14 as an antibacterial product, as odour-reducing product, as wound healing, styptic and blood coagulation promoting product, in non-woven products and/or as a filling fibre.

16. A moulded body according to any of the claims 12 to 14, in particular with a chitosan content of 0.2 w% to 1 w%, for the specific use as wound healing product, in particular as a product promoting the cell proliferation in the regenerating epidermis.

## Revendications

1. Procédé pour le traitement d'un corps moulé cellulosique, le corps moulé étant mis en contact avec une solution acide d'un chitosane, **caractérisé en ce que** le chitosane présente un degré de désacétylation d'au moins 80 %, une teneur en azote d'au moins 7 % en poids, de préférence d'au moins 7,5 % en poids, une moyenne en poids de la masse molaire Mw (D) de 10 kDa à 1000 kDa, de préférence de 10 kDa à 160 kDa et une viscosité dans une solution à 1 % en poids d'acide acétique à 1 % en poids à 25 °C de 1000 mPas ou moins, de préférence de 400 mPas ou moins, particulièrement préférentiellement de 200 mPas ou moins.

2. Procédé selon la revendication 1, **caractérisé en ce que** la teneur en chitosane dans la solution atteint 0,1 à 10 % en poids, de préférence 1 à 4 % en poids.

3. Procédé selon la revendication 1 ou 2, **caractérisé en ce que** le corps moulé se présente sous la forme de fibres.

4. Procédé selon la revendication 3, **caractérisé en ce que** les fibres sont des fibres de Lyocell, des fibres de Modal, des fibres de Polynosic et/ou des fibres de Viscose.

5. Procédé selon la revendication 3 ou 4, **caractérisé en ce que** les fibres se présentent sous une forme déjà sèche lors du traitement, en particulier sous la forme d'un composant d'un article textile, de préférence d'un fil, d'un tissu, d'un tricot, d'un vêtement fabriqué à partir de ceux-ci, ou d'un article non tissé.

6. Procédé selon la revendication 3 ou 4, **caractérisé en ce que** les fibres se présentent lors du traitement sous une forme nullement séchée.

7. Procédé selon la revendication 6, **caractérisé en ce que** les fibres se présentent sous la forme d'un matelas de fibres.

8. Procédé selon la revendication 6 ou 7, **caractérisé en ce que** les fibres présentent avant le traitement une humidité résiduelle de 50 % à 500 %.

9. Procédé selon l'une des revendications précédentes, **caractérisé en ce que** le corps moulé est soumis après traitement avec la solution à un traitement à la vapeur brûlante.

10. Procédé selon l'une des revendications précédentes, **caractérisé en ce que** le chitosane est dissous dans un acide inorganique ou organique pour la préparation de la solution.

11. Procédé selon la revendication 10, **caractérisé en ce que** l'acide est choisi dans le groupe constitué des acides mono-, di- ou tricarboxyliques ayant de 1 à 30 atomes de C, de préférence l'acide lactique, l'acide acétique, l'acide formique, l'acide propionique, l'acide glycolique, l'acide citrique, l'acide oxalique et des mélanges de ceux-ci.

12. Corps moulé pouvant être obtenu par un procédé selon l'une des revendications 1 à 11.

13. Corps moulé selon la revendication 12 sous la forme de fibres, de préférence de fibres de Lyocell, de fibres de Modal, de fibres de Polynosic et/ou de fibres de Viscose.

14. Corps moulé selon l'une des revendications 12 ou 13, **caractérisé par** une teneur en chitosane de 0,1 % en poids et plus, de préférence de 0,2 % en poids à 1 % en poids, particulièrement préférentiellement de 0,4 à 0,6 % en poids.

15. Utilisation d'un corps moulé selon l'une des revendications 12 à 14 comme produit antibactérien, produit inhibant les odeurs, produit cicatrisant, hémostatique et coagulant, dans des produits non tissés et/ou comme fibres de remplissage.

16. Corps moulé selon l'une des revendications 12 à 14, ayant notamment une teneur en chitosane de 0,2 % en poids à 1 % en poids, pour une utilisation spécifique comme produit cicatrisant, notamment favorisant la prolifération cellulaire dans l'épiderme en cours de régénération.
